(19) Europäisches Patentamt
European Patent Office
Office européen des brevets

(11) Numéro de publication: **0 139 584**
**B1**

# (12) FASCICULE DE BREVET EUROPEEN

(45) Date de publication du fascicule du brevet: **18.05.88**

(21) Numéro de dépôt: **84402019.8**

(22) Date de dépôt: **08.10.84**

(51) Int. Cl.$^4$: **C 07 D 471/06,**
C 07 D 487/06,
A 61 K 31/435, A 61 K 31/55
// (C07D471/06, 221:00,
209:00),(C07D487/06, 223:00,
209:00)

## (54) Dérivés d'imidazoline, leur préparation et leur application en thérapeutique.

(30) Priorité: **17.10.83 FR 8316474**
**09.02.84 FR 8401997**
**04.06.84 FR 8408726**

(43) Date de publication de la demande:
**02.05.85 Bulletin 85/18**

(45) Mention de la délivrance du brevet:
**18.05.88 Bulletin 88/20**

(84) Etats contractants désignés:
**AT BE CH DE FR GB IT LI LU NL SE**

(56) Documents cités:
**EP-A-0 071 368**
**GB-A-1 394 373**
**GB-A-1 394 374**

**JOURNAL OF HETEROCYCLIC CHEMISTRY, vol. 11, no. 3, June 1974, pages 387-393, Orem, Utah, US; E.A. STECK et al.: "Some 5,6-dihydro-4H-pyrrolo3,2,1-i,jr quinolines"**
**Chimie Thérapeutique 7 (1972), p. 345-349**

(73) Titulaire: **SYNTHELABO**
**58, rue de la Glacière**
**F-75621 Paris Cedex 13 (FR)**

(72) Inventeur: **Bigg, Dennis M.**
**5, Parc de Diane**
**F-78350 Jouy en Josas (FR)**
Inventeur: **Morel, Claude M.**
**25, rue Gabriel Péri Cressely**
**F-78470 Magny les Hameaux (FR)**
Inventeur: **Sevrin, Mireille**
**73, rue Raymond Losserand**
**F-75014 Paris (FR)**

(74) Mandataire: **Ludwig, Jacques et al**
**SYNTHELABO Service Brevets**
**58, rue de la Glacière**
**F-75621 Paris Cedex 13 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 139 584 B1

**Description**

La présente invention a pour objet des dérivés d'imidazoline, leur préparation et leur application en thérapeutique.

Les composés de l'invention répondent à la formule générale I donnée dans le schéma de la page suivante, formule dans laquelle

n représente le nombre 1 ou 2,

R représente un atome d'hydrogène ou un groupe alkyle en $C_1$—$C_3$ ou un groupe allyle, et

X représente un atome d'hydrogène ou d'halogène ou un groupe méthyle ou méthoxy.

Les composés de l'invention comportent dans leur molécule un atome de carbone asymétrique. Ils peuvent donc se présenter sous forme d'énantiomères ou de mélanges de ces derniers. Ces diverses formes font partie de l'invention.

Les composés peuvent former des sels d'addition avec des acides. Ces sels, en particulier s'ils sont pharmaceutiquement acceptables, font également partie de l'invention.

Des dérivés de pyrrolo[3,2,1-*ij*]quinolines sont connus en tant que tels grâce à E. A. Steck et coll., J. Het. Chem., *11*(3), 387—393, (1974). Les brevets GB—1.394.373 et 1.394.374 décrivent des composés ayant ce même squelette et utilisables comme fongicides. Enfin la demande de brevet EP—0 071 368 décrit des (dihydro-4,5 1*H*-imidazolyl-2)-2 dihydro-2,3 benzofurannes comme antagonistes des récepteurs $\alpha_2$-adrénergiques.

Les composés de l'invention peuvent être préparés selon le schéma donné à la page suivante.

Les esters de départ (II) dans lesquels n vaut 1 et R' est un radical $C_1$—$C_4$ alkyle peuvent être préparés selon la méthode décrite par E. A. Steck et coll. (op. cit.) pour l'ester méthylique (R = $CH_3$).

Les esters de départ (II) dans lesquels n vaut 2 peuvent être préparés de manière analogue à partir de la tétrahydro-2,3,4,5 1*H*-benzazépine décrite par P. E. Reyl et J. L. A. Rollet dans le brevet français 1 473 839 [cf. C.A. *68*, (1968), 78164e].

Schéma

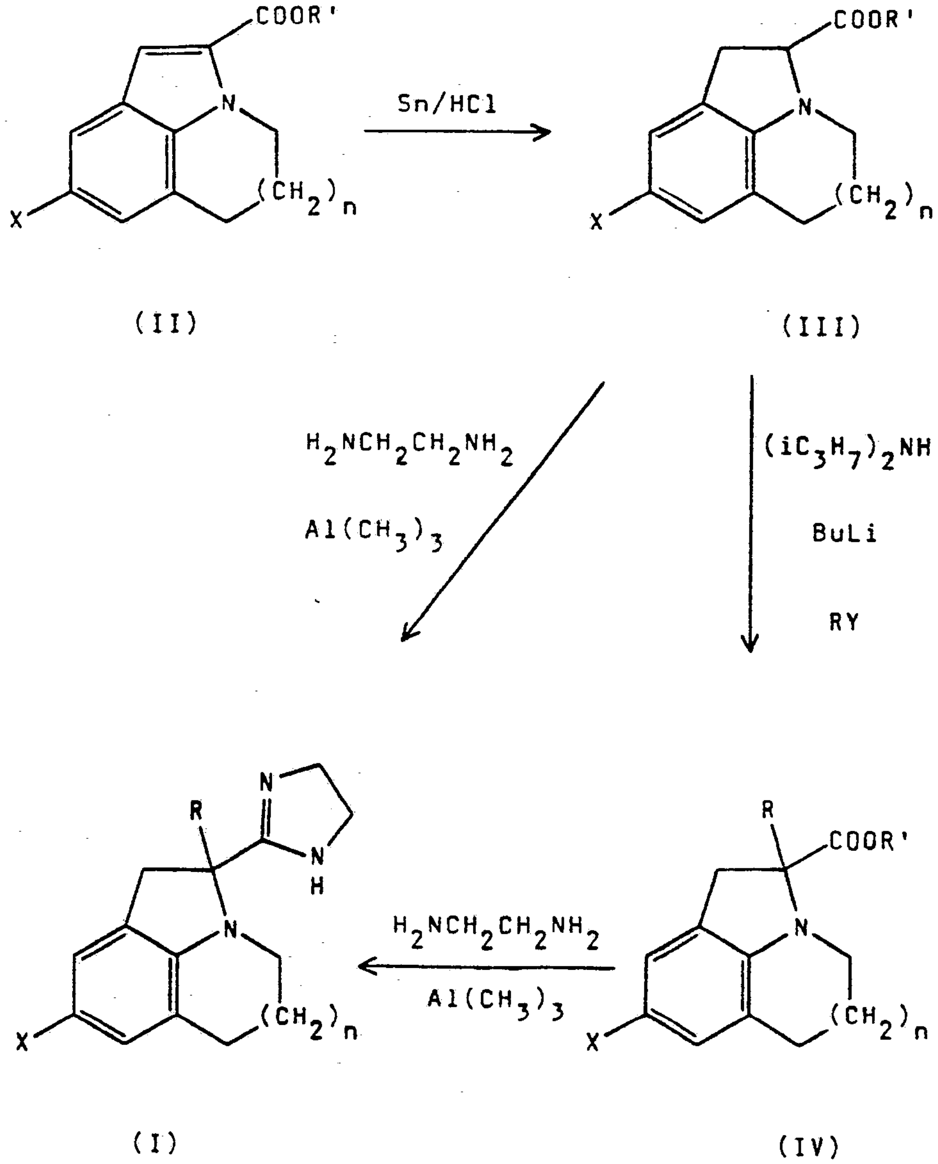


Selon l'invention on réduit l'ester de formule générale II, dans laquelle R' est un groupe alkyle inférieur, en particulier un groupe éthyle, par l'étain en présence de chlorure d'hydrogène pour aboutir à l'ester de formule générale III.

Si l'on veut obtenir le composé de formule générale I dans laquelle R désigne l'hydrogène, on peut alors soumettre directement l'ester (III) à l'action de l'éthylènediamine en présence de triméthylaluminium, pour former le cycle d'imidazoline.

Sinon, on fait précéder cette étape par une alkylation, effectuée par exemple par action d'un composé de formule générale RY (R étant tel que défini ci-dessus et Y étant un groupe labile tel qu'un atome d'iode ou de brome) sur le dérivé lithié préparé in situ au moyen de diisopropylamidure de lithium (butyllithium + diisopropylamine).

L'ester alkylé de formule générale IV est ensuite mis à réagir avec l'éthylènediamine, comme indiqué ci-dessus à propos du composé de formule générale III.

Les exemples suivants illustrent l'invention. Les analyses et les spectres IR et RMN confirment la structure des composés obtenus.

Exemple 1

(Dihydro-4,5 1*H*-imidazolyl-2)-2 tétrahydro-1,2,5,6 4*H*-pyrrolo[3,2,1-*ij*]quinoline et son fumarate

a) Dans un ballon tricol de 1 l, muni d'un réfrigérant à reflux et d'une arrivée de gaz chlorhydrique et contenu dans un bain de carboglace et d'alcool isopropylique, on introduit 24,7 g (0,108 mole) de dihydro-5,6 4*H*-pyrrolo[3,2,1-*ij*]quinolinecarboxylate-2 d'éthyle et 210 ml d'éthanol absolu. On condense le gaz chlorhydrique jusqu'à dissolution complète de l'ester de départ à la température de −20°C. On ajoute alors,

en une seule fois, 38,3 g (0,323 at.-g) d'étain en grenailles. On agite le mélange réactionnel à la température ambiante pendant 17 heures.

On concentre la solution et reprend l'huile et le solide formés par 750 ml d'éthanol absolu.

Après avoir ramené le pH à 9—10 par addition d'ammoniac gazeux, on essore les sels d'étain formés.

On évapore à sec le filtrat éthanolique, le reprend avec de l'eau glacée et extrait avec de l'éther éthylique. On sèche sur sulfate de sodium et évapore à sec sous vide. On obtient le tétrahydro-1,2,5,6 4*H*-pyrrolo[3,2,1-*ij*]quinoline carboxylate-2 d'éthyle sous forme d'une huile.

b) Sous argon, on introduit successivement dans le ballon 21 ml de toluène, 13,7 ml (0,032 mole) de triméthylaluminium à 25,2% dans l'hexane. On refroidit le mélange dans de la glace et ajoute 2,1 ml (0,032 mole) d'éthylènediamine dans 6 ml de toluène.

On agite le mélange 5 minutes puis ajoute, à 50°C, 4,6 g (0,02 mole) de tétrahydro-1,2,5,6 4*H*-pyrrolo[3,2,1-*ij*]quinolinecarboxylate-2 d'éthyle obtenu précédemment dans 18 ml de toluène.

On chauffe le mélange réactionnel à la température du reflux. On élimine 18 ml de solvant et maintient le mélange à la température de reflux pendant 12 heures.

On refroidit le mélange réactionnel et ajoute 13,2 ml d'eau; on extrait le mélange à l'acétate d'éthyle, lave la phase organique avec une solution de chlorure de sodium et sèche sur sulfate de sodium.

Après filtration et concentration on obtient le produit qui est la (dihydro-4,5 1*H*-imidazolyl-2)-2 tétrahydro-1,2,5,6 4*H*-pyrrolo[3,2,1-*ij*]quinoline.

On transforme directement ce composé en fumarate, en faisant réagir 4,2 g (0,018 mole) du composé dissous dans 50 ml d'éthanol avec 1,97 g (0,017 mole) d'acide fumarique dissous dans 100 ml d'éthanol.

On agite pendant 15 minutes, évapore à sec, reprend avec de l'acétone et filtre le produit. Après recristallisation dans de l'éthanol le composé fond à 186—188°C.

Exemple 2

Méthyl-2 (dihydro-4,5 1*H*-imidazolyl-2)-2 tétrahydro-1,2,5,6 4*H*-pyrrolo[3,2,1-*ij*]quinoline et son fumarate

a) On opère comme à l'exemple 1a).

b) Dans un ballon de Keller de 100 ml muni d'une agitation magnétique, d'un thermomètre, d'une arrivée d'argon, d'une ampoule à introduction et placé dans un bain froid, on introduit 1,7 ml (0,012 mole) de diisopropylamine et 10 ml de tétrahydrofuranne, et on le refroidit à −75°C.

On ajoute alors, en l'espace de 15 minutes, 7,5 ml d'une solution 1,6 M de butyllithium dans l'hexane.

On agite le mélange pendant une heure à −75°C puis, en l'espace de 10 minutes, on ajoute 2,1 g (0,009 mole) de l'huile précédemment obtenue en solution dans 7,5 ml de tétrahydrofuranne.

On agite encore pendant une heure puis, en l'espace de 10 minutes, on ajoute 7,1 g, soit 3,1 ml (0,05 mole) de iodure de méthyle en solution dans 5 ml de tétrahydrofuranne.

On agite le mélange pendant une heure à −75°C, puis on le laisse revenir à environ 0°C.

On le verse alors sur un mélange d'eau et de glace, on l'extrait à l'éther éthylique en présence d'une solution saturée de chlorure de sodium, on lave la phase organique à l'eau, on la sèche, et on l'évapore sous vide au bain-marie. Il reste une huile jaune qu'on purifie par passage sur une colonne de silice en éluant avec du chlorure de méthylène. On obtient finalement une huile jaune qui cristallise au froid.

c) Dans un ballon de Keller de 50 ml muni d'une agitation magnétique, d'un réfrigérant à reflux surmontant un appareil de Dean-Stark, d'un thermomètre, d'une ampoule à introduction et d'une arrivée d'argon, on introduit 10 ml de toluène et 5,4 ml (0,013 mole) de triméthylaluminium en solution à 25% dans l'hexane, et on refroidit le mélange par un bain de glace.

On ajoute alors 0,9 ml (0,013 mole) d'éthylènediamine en solution dans 3 ml de toluène.

On agite 10 minutes à 0°C, puis on chauffe à 50°C et on ajoute 2 g (0,0082 mole) de l'huile précédemment obtenue en solution dans 10 ml de toluène.

On chauffe ensuite au reflux pendant 15 heures, puis on refroidit le mélange à −10°C et on l'hydrolyse avec 5,4 ml d'eau.

On l'agite pendant 10 minutes, on ajoute de l'acétate d'éthyle, on sépare la matière minérale, on lave la phase organique avec une solution saturée de chlorure de sodium, on la sèche sur sulfate de sodium, et on la concentre. Il reste un composé huileux, jaune foncé, qui cristallise après quelques heures.

On en dissout 2,05 g dans 25 ml d'éthanol et on ajoute une solution de 0,8 g d'acide fumarique dans 50 ml d'éthanol. On agite le mélange, on l'évapore, on le lave à l'acétone et on le recristallise dans le méthanol. Après séchage sous vide, le fumarate fond à 184,5—186°C.

Exemple 3

(Dihydro-4,5 1*H*-imidazolyl-2)-2 hexahydro-1,2,4,5,6,7 azépino[3,2,1-*hi*]indole et son fumarate

a) Dans un ballon tricol de 1 l muni d'une agitation magnétique, d'un thermomètre, d'une ampoule à introduction et placé dans un bain de glace on introduit 42,3 g (0,287 mole) de tétrahydro-2,3,4,5 1*H*-benzazépine et 240 ml d'acide sulfurique à 25%. On laisse le mélange refroidir à 0°C puis on introduit goutte à goutte, en 30 minutes, 20,7 g (0,3 mole) de nitrite de sodium en solution dans 30 ml d'eau.

Il se forme un précipité qu'on agite pendant 2 heures, puis on extrait le mélange avec du chlorure de méthylène, on sépare la phase organique, on la lave, on la sèche et on l'évapore sous vide au bain-marie.

On recueille une huile qu'on utilise telle quelle dans l'étape suivante.

b) Dans un ballon tricol de 3 litres muni d'une agitation magnétique, d'un réfrigérant à reflux, d'une

garde à chlorure de calcium, d'un thermomètre, d'une ampoule à introduction et placé sous atmosphère d'argon, on introduit 11,7 g (0,308 mole) d'hydrure d'aluminium et de lithium en poudre et 400 ml d'éther éthylique puis, à la suspension obtenue, on ajoute une solution de 49,3 g (0,279 mole) de l'huile précédemment obtenue dans 600 ml d'éther éthylique. L'addition dure une heure, et on maintient la température à environ 20°C par un bain glacé.

Après 20 heures de réaction il se forme un léger précipité que l'on dissout en ajoutant 500 ml de tétrahydrofuranne. Puis on chauffe le mélange au reflux pendant 6 heures, avant de laisser reposer une nuit à température ambiante.

Après avoir placé le mélange dans un bain de glace, on l'hydrolyse successivement par 8 ml d'eau, 8 ml d'hydroxyde de sodium 1N et 24 ml d'eau.

Après 1 heure d'agitation, on essore le précipité formé, on le lave avec du tétrahydrofuranne, on sèche le filtrat et on l'évapore sous vide au bain-marie. Il reste une huile rougeâtre qu'on utilise telle quelle dans l'étape suivante.

c) Dans un ballon de 500 ml muni d'une agitation magnétique, d'un réfrigérant à reflux et d'une garde à chlorure de calcium, on introduit successivement 23,8 g (0,147 mole) de l'huile précédemment obtenue, 230 ml d'éthanol absolu, 17,9 g (0,154 mole) de pyruvate d'éthyle et 15 gouttes d'acide acétique glacial.

On chauffe le mélange au reflux pendant 1 heure, puis on le verse dans un ballon de 2 litres contenant de l'éthanol saturé par du chlorure d'hydrogène gazeux.

On chauffe le mélange à ébullition pendant 2 heures, puis on l'évapore à sec sous vide et au bain-marie.

Il reste un résidu marron qu'on reprend avec du chlorure de méthylène, on lave la solution à l'eau, on la sèche et on l'évapore de nouveau. Il reste un produit résineux qu'on purifie par chromatographie sur silice en éluant avec du chlorure de méthylène. On recueille finalement une huile jaune clair assez mobile.

d) Dans un ballon tricol de 500 ml muni d'une agitation magnétique, d'une arrivée de chlorure d'hydrogène gazeux, d'un réfrigérant avec garde à chlorure de calcium, d'un thermomètre, et placé dans un bain de glace, on introduit 8 g (0,0329 mole) de l'ester précédemment obtenu et 70 ml d'éthanol.

On refroidit le mélange à −20°C et on condense le chlorure d'hydrogène à cette température jusqu'à obtenir une solution de couleur rouge-orange; le mélange double de volume.

On ajoute alors en une seule fois 11,9 g (0,1 at.-g) de grenaille d'étain, on retire le bain froid, et on agite le mélange à température ambiante.

Après 26 heures de réaction, on évapore le mélange sous vide au bain-marie, on le reprend avec 250 ml d'éthanol, on le refroidit à −10°C et on y fait barboter de l'ammoniac jusqu'à un pH de 9 à 10.

On essore les sels d'étain qui ont précipité, on les lave à l'éthanol glacé puis à l'éther, on évapore les phases organiques, on traite le résidu à l'eau glacée et on l'extrait avec de l'éther.

Après lavage et séchage de la phase éthérée, on l'évapore et on recueille une huile de couleur jaune.

e) Dans un ballon de Keller de 100 ml muni d'une agitation magnétique, d'un réfrigérant à reflux avec garde à chlorure de calcium, d'un thermomètre, d'une arrivée d'argon, d'une ampoule à introduction, et d'un appareil de Dean-Stark, on introduit successivement 11 ml de toluène et 6,85 ml (0,16 mole) d'une suspension de triméthylaluminium à 25,2% dans l'hexane.

On refroidit le mélange par un bain glacé, et on ajoute une solution de 1,05 ml (0,016 mole), soit 0,95 g, d'éthylènediamine dans 3 ml de toluène.

On agite le mélange pendant 5 minutes, puis on le chauffe; vers 50°C, on y ajoute une solution de 2,45 g (0,01 mole) de l'huile précédemment préparée dans 9 ml de toluène.

Ensuite on chauffe au reflux, en éliminant environ 8 ml de solvant jusqu'à obtenir une température intérieure de 110°C. Après 6 heures de chauffage, on refroidit le mélange à environ −15°C et on l'hydrolyse avec 6,6 ml d'eau.

On l'agite pendant 10 minutes, on l'extrait à l'acétate d'éthyle, on lave la phase organique trois fois avec une solution saturée de chlorure de sodium, on la sèche sur sulfate de sodium, on la filtre et on évapore le solvant. Il reste un solide qui fond à 138—140°C et dont on prépare directement le fumarate.

Pour cela on en dissout 1,9 g (0,0079 mole) dans 25 ml d'éthanol, on filtre et on mélange avec une solution filtrée de 0,8 g (0,0068 mole) d'acide fumarique dans 50 ml d'éthanol.

On agite le mélange pendant 15 minutes, on l'évapore à sec au bain-marie et sous vide, et on reprend le résidu avec de l'acétone. Il se forme des cristaux que l'on essore et rince à l'acétone. Après séchage sous vide et recristallisation dans l'alcool isopropylique, il reste des cristaux jaunes qui fondent entre 108 et 140°C.

### Exemple 4

(Dihydro-4,5 1*H*-imidazolyl-2)-2 méthyl-2 hexahydro-1,2,4,5,6,7 azépino[3,2,1-*hi*]indole et son fumarate

a) Dans un ballon de Keller de 100 ml muni d'une agitation magnétique, d'un thermomètre, d'une arrivée d'argon, d'une ampoule à introduction et d'un bain réfrigérant, on introduit, sous argon, 2,3 ml (0,016 mole) de diisopropylamine et 15 ml de tétrahydrofuranne, on refroidit cette solution vers −75°C puis, en 15 minutes, on introduit 10 ml (0,016 mole) de butyllithium en solution 1,6 M dans l'hexane.

On maintient le mélange à −75°C pendant 1 heure puis, en l'espace de 10 minutes, on ajoute 3,2 g (0,013 mole) de l'ester hydrogéné obtenu comme indiqué à l'exemple 3 d), en solution dans 10 ml de tétrahydrofuranne.

On agite encore 1 heure à −75°C, puis on ajoute en 10 minutes 4,5 ml (0,072 mole), soit 10,3 g de iodométhane en solution dans 7 ml de tétrahydrofuranne.

On agite encore 1 heure 30 à −75°C, puis on laisse reposer le mélange quelques heures au réfrigérateur, toujours sous atmosphère d'argon.

On le verse ensuite sur de l'eau glacée, on l'extrait avec de l'éther éthylique en présence d'une solution saturée de chlorure de sodium.

Après lavage, séchage et évaporation de la phase organique, il reste une huile que l'on purifie par chromatographie sur silice en éluant avec du chlorure de méthylène.

b) Dans un ballon de Keller de 50 ml muni d'une agitation magnétique, d'un réfrigérant à reflux avec garde à chlorure de calcium, d'un thermomètre, d'une arrivée d'argon, d'une ampoule à introduction et d'un appareil de Dean Stark, on introduit, sous argon, 11 ml de toluène et 6,85 ml (0,016 mole) d'une suspension de triméthylaluminium à 25,2% dans l'hexane.

On refroidit le mélange, puis on ajoute 1,05 ml (0,016 mole) soit 0,95 g d'éthylènediamine, en solution dans 3 ml de toluène.

Tout en agitant le mélange on le chauffe et, vers 50°C, on y ajoute une solution de 2,5 g (0,0096 mole) de l'huile précédemment préparée dans 9 ml de toluène.

On chauffe le mélange au reflux en éliminant environ 10 ml de solvant pour obtenir une température intérieure de 110°C. Après 8 heures de chauffage on refroidit le mélange à environ −15°C et on l'hydrolyse avec 6,6 ml d'eau.

On l'agite pendant 10 minutes, puis on l'extrait à l'acétate d'éthyle, on lave la phase organique avec une solution saturée de chlorure de sodium, on la sèche, on la filtre et on l'évapore. Il reste une huile jaune dont on prépare directement le fumarate.

Pour cela on dissout 2,5 g (0,0096 mole) de cette huile dans 25 ml d'éthanol, on filtre la solution, et on la mélange avec une solution filtrée de 0,9 g (0,008 mole) d'acide fumarique dans 50 ml d'éthanol.

Après 15 minutes d'agitation, on évapore le mélange sous vide au bain-marie, et on reprend le résidu par de l'acétone. On essore et on rince les cristaux formés, on les sèche sous vide et on les recristallise dans de l'éthanol. Le produit fond à 212—213°C.

## Exemple 5

(Dihydro-4,5 1*H*-imidazolyl-2)-2 fluoro-8 tétrahydro-1,2,5,6 4*H*-pyrrolo[3,2,1-*ij*]quinoline et son fumarate

a) Dans un ballon tricol de 1 l, muni d'un réfrigérant à reflux, d'une agitation magnétique, d'un thermomètre et d'une arrivée de gaz chlorhydrique et placé dans un bain de carboglace et d'alcool isopropylique, on introduit 18,52 g (0,075 mole) de dihydro-5,6 fluoro-8 4*H*-pyrrolo[3,2,1-*ij*]quinoline-carboxylate-2 d'éthyle et 160 ml d'éthanol. On refroidit le mélange à −60°C, puis on condense le gaz chlorhydrique jusqu'à dissolution complète de l'ester de départ. On ajoute alors 28,72 g (0,24 at.-g) de grenaille d'étain et on agite le mélange pendant 20 heures à 20°C.

On évapore le solvant sous vide, on reprend le résidu avec 500 ml d'éthanol et on sature le mélange par de l'ammoniac jusqu'a pH > 9.

On filtre la suspension et on évapore le solvant de filtrat. On ajoute 200 ml d'eau et on extrait avec 600 ml d'éther, on lave la phase éthérée avec de l'eau, puis avec de l'eau saturée de chlorure de sodium, on la sèche sur sulfate de magnésium, et on évapore l'éther sous vide. On obtient le tétrahydro-1,2,5,6 fluoro-8 4*H*-pyrrolo[3,2,1-*ij*]quinolinecarboxylate-2 d'éthyle sous forme d'une huile.

b) Dans un ballon tricol de 150 ml muni d'une agitation magnétique, d'un réfrigérant et d'une arrivée d'argon, on introduit 21 ml de toluène et on ajoute 25 g (13,7 ml, soit 0,032 mole) de triméthylaluminium à 25,2% dans l'hexane.

On refroidit la solution à −10°C et on ajoute 1,92 g (0,032 mole) d'éthylènediamine en solution dans 6 ml de toluène puis, à 20°C on ajoute 3,73 g (0,015 mole) de l'ester précédemment obtenu en solution dans 20 ml de toluène. On agite le mélange à 110°C pendant 4 heures, on le refroidit à −10°C et on ajoute lentement 10 ml d'eau et 50 ml d'acétate d'éthyle.

On agite à 20°C pendant 30 minutes, on filtre le mélange, on sépare la phase organique, on la lave et on la sèche, et on évapore l'acétate d'éthyle.

On reprend la base ainsi obtenue avec 20 ml d'éthanol et on ajoute une solution de 0,7 g d'acide fumarique dans 20 ml d'éthanol. On agite le tout pendant 15 minutes et on chasse l'alcool sous vide. Le fumarate obtenu fond à 182—185°C.

Le tableau suivant illustre les autres composés préparés selon l'invention.

TABLEAU

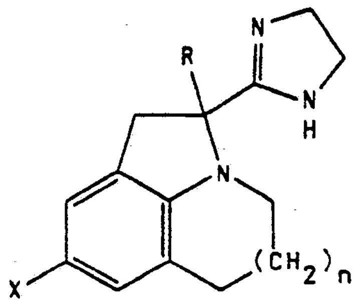

(I)

| Composé | Exemple | R | X | n | F(°C) | Base/sel(*) |
|---|---|---|---|---|---|---|
| 1 | 1 | H | H | 1 | 186—188 | 08 |
| 2 | 2 | $—CH_3$ | H | 1 | 184,5—186 | 08 |
| 3 | | $—CH_2CH_3$ | H | 1 | 182—184 | 08 |
| 4 | | $—CH_2CH_2CH_3$ | H | 1 | 218—220 | 08 |
| 5 | | $—CH_2CH{=}CH_2$ | H | 1 | 195—197 | 08 |
| 6 | 3 | H | H | 2 | 108—140 | 08 |
| 7 | 4 | $—CH_3$ | H | 2 | 212—213 | 08 |
| 8 | | $—CH_2CH_2CH_3$ | H | 2 | 197—199 | 08 |
| 9 | 5 | H | F | 1 | 182—185 | 08 |
| 10 | | $—CH_3$ | F | 1 | 199—202 | 08 |
| 11 | | $—CH_2CH_2CH_3$ | F | 1 | 210—212 | 08 |
| 12 | | $—CH_2CH_2CH_3$ | $CH_3$ | 1 | 198—200 | 08 |
| 13 | | H | $CH_3$ | 1 | 173—175 | 08 |
| 14 | | $—CH_3$ | $CH_3$ | 1 | 154—158 | 08 |
| 15 | | H | Cl | 1 | 195—196 | 08 |
| 16 | | $—CH_3$ | Cl | 1 | >260 | 10 |
| 17 | | H | $OCH_3$ | 1 | 144—147 | 08 |

(*): 08: fumarate
10: chlorhydrate

Les composés de l'invention ont été soumis à des essais pharmacologiques qui ont montré leur intérêt en tant que $\alpha_2$-antagonistes.

A cet effet les composés ont été étudiés dans le test de potentialité et de sélectivité des antagonistes à l'égard des récepteurs $\alpha_2$ in vitro.

La détermination de la valeur $pA_2$ à l'égard des effets inhibiteurs de la clonidine, $\alpha_2$-agoniste bien connu, a eu lieu sur le vas deferens du rat stimulé à une fréquence de 0,1 Hz en présence de 300 nM de prazosine et de 1 µM de cocaïne, selon la méthode décrite par G. M. Drew (European Journal of Pharmacology, 42, (1977) 123—130.

Les $pA_2$ des composés de l'invention vont de 6,0 à 9,0.

Les composés de l'invention sont des $\alpha_2$-antagonistes qui peuvent être utilisés pour le traitement de la dépression (soit seuls, soit en association avec des produits qui inhibent les mécanismes de captation neuronale), le traitement de l'hypotension, le traitement de l'ileum paralytique post-opératoire, le

traitement de l'asthme et de l'obésité.

Les compositions pharmaceutiques de l'invention peuvent être sous une forme appropriée pour l'administration par voie orale, rectale ou parentérale, par exemple sous la forme de capsules, comprimés, granulés, gélules ou solutés liquides, sirops ou suspensions buvables, et contenir les excipients appropriés.

La posologie quotidienne peut aller de 0,1 à 20 mg/kg par voie orale.

**Revendications pour les Etats contractants: BE CH DE FR GB IT LI LU NL SE**

1. Composés sous forme d'énantiomères ou de leurs mélanges, répondant à la formule générale I

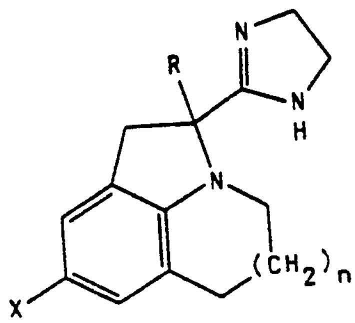
(I)

dans laquelle

n représente le nombre 1 ou 2,

R représente un atome d'hydrogène ou un groupe alkyle en $C_1$—$C_3$ ou un groupe allyle, et

X représente un atome d'hydrogène ou d'halogène ou un groupe méthyle ou méthoxy,

ainsi que leurs sels d'addition à des acides pharmaceutiquement acceptables.

2. Procédé de préparation des composés selon la revendication 1, caractérisé en ce qu'on réduit le composé de formule générale II

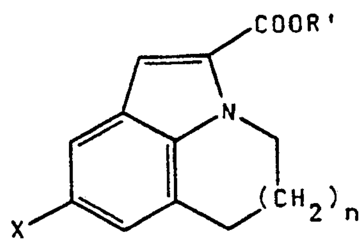
(II)

dans laquelle n et X sont tels que définis dans la revendication 1 et R' est un groupe alkyle inférieur, pour obtenir un composé de formule générale III

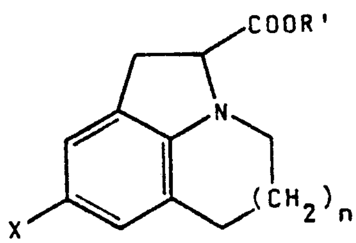
(III)

puis, si l'on désire obtenir un composé final dans la formule générale I duquel R représente un groupe alkyle en $C_1$—$C_3$ ou un groupe allyle, on soumet le composé (III) ainsi obtenu à une alkylation en le faisant réagir avec le diisopropylamidure de lithium suivi d'un composé de formule générale RY dans laquelle R est un groupe alkyle en $C_1$—$C_3$ ou un groupe allyle et Y est un groupe labile, pour obtenir un composé de formule générale IV

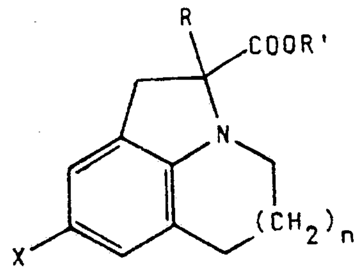

(IV)

et enfin on fait réagir le composé de formule générale III ou de formule générale IV avec de l'éthylène-diamine en présence de triméthylaluminium.

3. Médicament caractérisé en ce qu'il est constitué par un composé selon la revendication 1.

4. Composition pharmaceutique caractérisée en ce qu'elle contient un composé selon la revendication 1, en association avec un excipient.

**Revendication pour l'Etat contractant: AT**

Procédé de préparation de composés répondant à la formule générale I

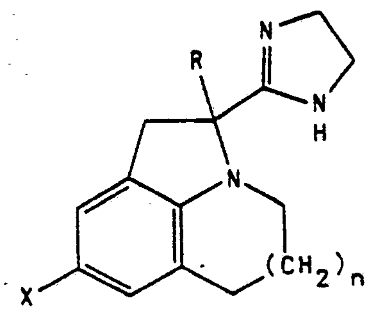

(I)

dans laquelle

n représente le nombre 1 ou 2,

R représente un atome d'hydrogène ou un groupe alkyle en $C_1$—$C_3$ ou un groupe allyle, et

X représente un atome d'hydrogène ou d'halogène ou un groupe méthyle ou méthoxy,

procédé caractérisé en ce qu'on réduit le composé de formule générale II

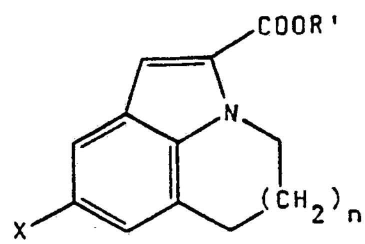

(II)

dans laquelle n et X sont tels que définis ci-dessus et R' est un groupe alkyle inférieur, pour obtenir un composé de formule générale III

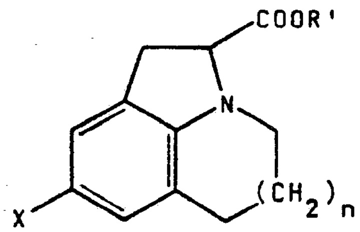

(III)

puis, si l'on désire obtenir un composé final dans la formule générale I duquel R représente un groupe alkyle en $C_1$—$C_3$ ou un groupe allyle, on soumet le composé (III) ainsi obtenu à une alkylation en le faisant réagir avec le diisopropylamidure de lithium suivi d'un composé de formule générale RY dans laquelle R est un groupe alkyle en $C_1$—$C_3$ ou un groupe allyle et Y est un groupe labile, pour obtenir un composé de formule générale IV

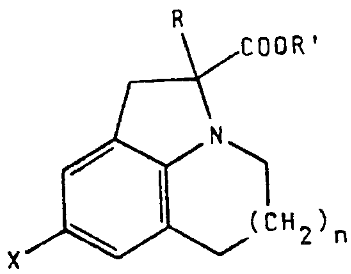

(IV)

et enfin on fait réagir le composé de formule générale III ou de formule générale IV avec de l'éthylènediamine en présence de triméthylaluminium.

**Patentansprüche für die Vertragsstaaten: BE CH DE FR GB IT LI LU NL SE**

1. Verbindungen in Form der Enantiomeren oder deren Mischungen der allgemeinen Formel I

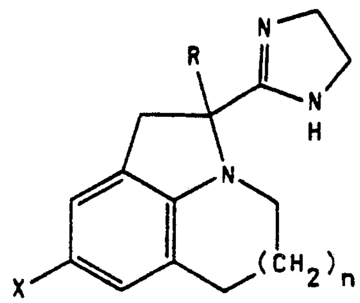

(I)

in welcher n für die Zahl 1 oder 2, R für ein Wasserstoffatom oder eine $C_1$—$C_3$-Alkyl- oder eine Allylgruppe und X für ein Wasserstoff- oder ein Halogenatom oder eine Methyl- oder Methoxygruppe steht, sowie deren Additionssalze mit pharmazeutisch verwendbaren Säuren.

2. Verfahren zur Herstellung von Verbindungen nach Anspruch 1, dadurch gekennzeichnet, daß man die Verbindung der allgemeinen Formel II

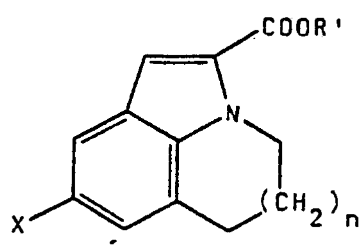

(II)

in welcher n und X die in Anspruch 1 genannte Bedeutung haben und R' für eine niedrige Alkylgruppe steht, zur Gewinnung einer Verbindung der allgemeinen Formel III

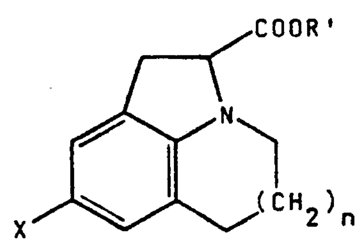

(III)

reduziert, dann für den Fall, daß eine Endverbindung hergestellt werden soll, in deren allgemeiner Formel I R für eine $C_1$—$C_3$-Alkyl- oder eine Allylgruppe steht, die so erhaltene Verbindung (III) einer Alkylierung durch Reaktion mit Lithiumdiisopropylamid und anschließend mit einer Verbindung der allgemeinen Formel RY, in welcher R für eine $C_1$—$C_3$-Alkyl- oder eine Allylgruppe und Y für eine abspaltbare Gruppe steht, unterwirft, um eine Verbindung der allgemeinen Formel IV

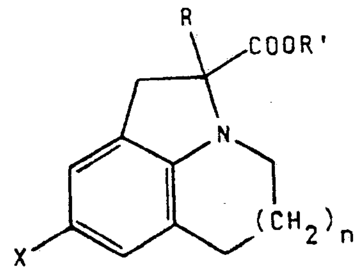

(IV)

zu erhalten, und schließlich die Verbindung der allgemeinen Formel III oder der allgemeinen Formel IV mit Ethylendiamin in Gegenwart von Trimethylaluminium zur Reaktion bringt.

3. Heilmittel, dadurch gekennzeichnet, daß es aus einer Verbindung nach Anspruch 1 besteht.

4. Pharmazeutische Zusammensetzung, dadurch gekennzeichnet, daß sie eine Verbindung nach Anspruch 1 in Kombination mit einem Bindemittel enthält.

**Patentanspruch für den Vertragsstaat: AT**

Verfahren zur Herstellung von Verbindungen der allgemeinen Formel I

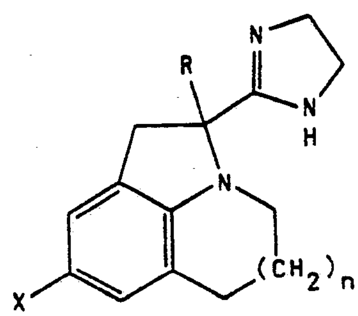

(I)

in welcher n für die Zahl 1 oder 2, R für ein Wasserstoffatom oder eine $C_1$—$C_3$-Alkyl- oder eine Allylgruppe und X für ein Wasserstoff- oder ein Halogenatom oder eine Methyl- oder Methoxygruppe steht, dadurch gekennzeichnet, daß man die Verbindung der allgemeinen Formel II

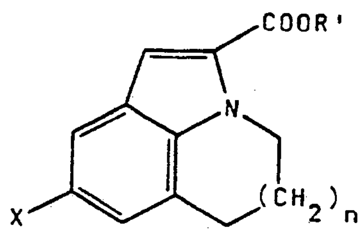

(II)

in welcher n und X die oben genannte Bedeutung haben und R′ für eine niedrige Alkylgruppe steht, zur Gewinnung einer Verbindung der allgemeinen Formel III

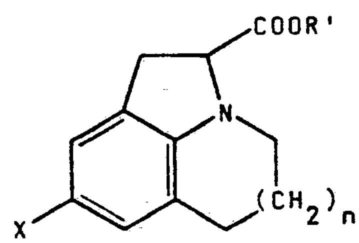

(III)

reduziert, dann für den Fall, daß eine Endverbindung hergestellt werden soll, in deren allgemeiner Formel I R für eine $C_1$—$C_3$-Alkyl- oder eine Allylgruppe steht, die so erhaltene Verbindung (III) einer Alkylierung durch Reaktion mit Lithiumdiisopropylamid und anschließend mit einer Verbindung der allgemeinen Formel RY, in welcher R für eine $C_1$—$C_3$-Alkyl- oder eine Allylgruppe und Y für eine abspaltbare Gruppe steht, unterwirft, um eine Verbindung der allgemeinen Formel IV

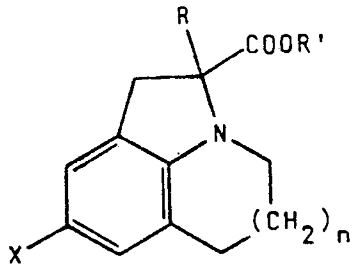

(IV)

zu erhalten, und schließlich die Verbindung der allgemeinen Formel III oder der allgemeinen Formel IV mit Ethylendiamin in Gegenwart von Trimethylaluminium zur Reaktion bringt.

**Claims for the Contracting States: BE CH DE FR GB IT LI LU NL SE**

1. Compounds in the form of enantiomers or mixtures thereof, corresponding to the general formula I

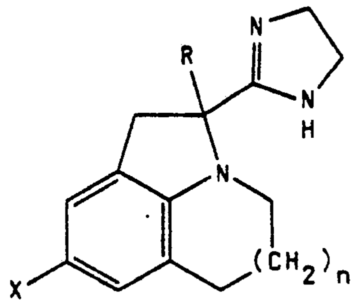

(I)

in which
n denotes the number 1 or 2,
R denotes a hydrogen atom or a $C_1$—$C_3$ alkyl group or an allyl group, and
X denotes a hydrogen or halogen atom or a methyl or methoxy group,
and also their addition salts with pharmaceutically acceptable acids.

2. Process for the preparation of the compounds according to Claim 1, characterized in that the compound of general formula II

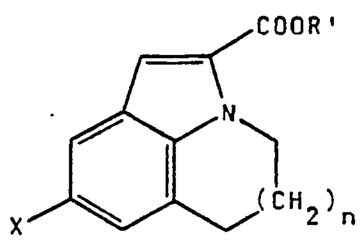

(II)

in which n and X are as defined in Claim 1 and R' is a lower alkyl group, is reduced to obtain a compound of general formula III

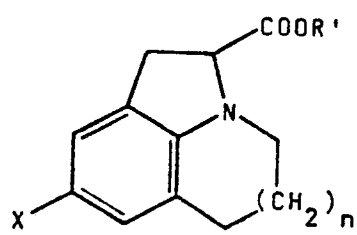

(III)

then, if it is desired to obtain a final compound of the general formula I in which R denotes a $C_1$—$C_3$ alkyl group or an allyl group, the compound (III) thus obtained is subjected to alkylation by reacting it with lithium diisopropylamide followed by a compound of general formula RY in which R is a $C_1$—$C_3$ alkyl group or an allyl group and Y is a labile group, to obtain a compound of general formula IV

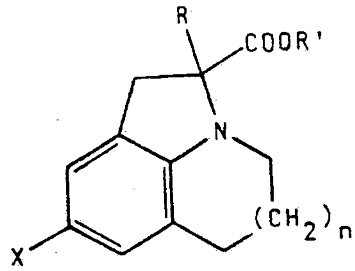

(IV)

and finally the compound of general formula III or IV is reacted with ethylenediamine in the presence of trimethylaluminium.

3. Drug, characterized in that it consists of a compound according to Claim 1.

4. Pharmaceutical composition, characterized in that it contains a compound according to Claim 1 in association with an excipient.

**Claim for the Contracting State: AT**

Process for the preparation of compounds corresponding to the general formula I

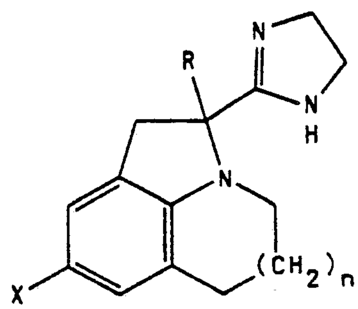

(I)

in which

n denotes the number 1 or 2,

R denotes a hydrogen atom or a $C_1$—$C_3$ alkyl group or an allyl group, and

X denotes a hydrogen or halogen atom or a methyl or methoxy group,

which process is characterized in that the compound of general formula II

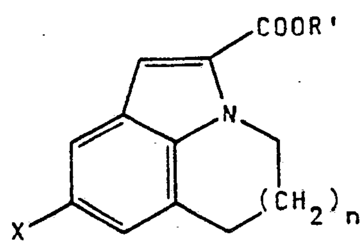

(II)

in which n and X are as defined above and R′ is a lower alkyl group, is reduced to obtain a compound of general formula III

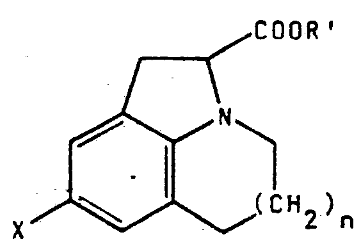

(III)

then, if it is desired to obtain a final compound of the general formula I in which R denotes a $C_1$—$C_3$ alkyl group or an allyl group, the compound (III) thus obtained is subjected to alkylation by reacting it with lithium diisopropylamide followed by a compound of general formula RY in which R is a $C_1$—$C_3$ alkyl group or an allyl group and Y is a labile group, to obtain a compound of general formula IV

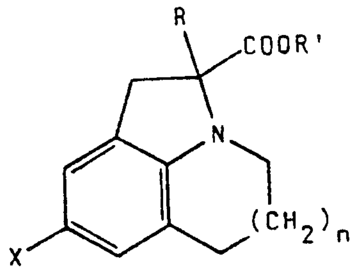

(IV)

and finally the compound of general formula III or IV is reacted with ethylenediamine in the presence of trimethylaluminium.